# EUROPEAN PATENT APPLICATION

(11) **EP 4 438 723 A1**
(43) Date of publication of application: **02.10.2024**
(21) Application number: 22919603.5
(22) Date of filing: 14.02.2022
(51) Int. Cl.: C12N 9/02, C12N 9/06, C12N 11/091

(54) **IMMOBILIZED ENZYME AND USE THEREOF IN CONTINUOUS PRODUCTION**

(30) Priority: 12.01.2022 CN 202210033913
(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); PAN, Long, Tianjin 300457 (CN); MA, Liteng, Tianjin 300457 (CN); CUI, Yuxia, Tianjin 300457 (CN); GAO, Yanyan, Tianjin 300457 (CN)
(74) Representative: Delbarba, Andrea
(86) International application number: PCT/CN2022/076232
(87) International publication number: WO 2023/133957

(57) **Abstract**

Provided are an immobilized enzyme and an application thereof in continuous production. The immobilized enzyme is a Polyethyleneimine (PEI)-modified immobilized enzyme, and includes: an enzyme, which includes Amine Dehydrogenase (AmDH) and/or Formate Dehydrogenase (FDH); and a carrier, which is a cyanuric chloride-activated amino carrier. The problem in the prior art of poor performance of an immobilized enzyme is solved, the catalytic activity and reusability of the immobilized enzyme are improved, and the immobilized enzyme is suitable for the field of enzyme immobilization.

## Description

### Technical Field

The present disclosure relates to the field of enzyme immobilization, and specifically, to an immobilized enzyme and an application thereof in continuous production.

### Background

Chiral amine is an important intermediate for the synthesis of chiral drugs, agro-drugs, fine chemicals etc. In recent years, in order to avoid many chemical synthesis processes of preparing key compounds, so as to meet requirements of green chemistry, the use of Amine Dehydrogenase (AmDH) is increasing. The AmDH refers to a class of enzymes that can catalyze the reduction of ketone compounds to the chiral amine. Different from transaminase, the AmDH uses an inexpensive ammonium salt as an ammonia source, and does not rely on expensive ammonia donors such as isopropylamine, alanine, etc. As a reduction process usually requires the delivery of hydrogen from reduced Nicotinamide Adenine Dinucleotide Phosphate (NADPH) or reduced Nicotinamide Adenine Dinucleotide (NADH), the AmDH is often used in combination with Formate Dehydrogenase (FDH) to reduce production costs. Compared with chemically-catalyzed synthesis, the AmDH is not only mild in reaction condition, but also possesses higher catalytic activity, selectivity, and substrate specificity. However, water-soluble free enzymes face many problems during production and application, such as poor stability, inability to be reused, and problems in product separation and purification.

A main task of enzyme immobilization is to select an appropriate immobilization method to design a biocatalyst that meets both the catalytic requirements (selectivity or stability) and the non-catalytic requirements (costs or separation processes) of an application. At present, covalent bonding is the most commonly used method for immobilizing enzymes in production applications, and commercially-available carriers used include amino carriers and epoxy carriers. Using amino-type resin as an example, the amino-type resin usually needs to be activated by a dual-functional crosslinking agent, and then bound to the enzymes through end functional groups of the crosslinking agent. The most commonly used activation method is to activate amino with glutaraldehyde, and then connect same with enzyme molecules using an active aldehyde group, so as to realize immobilization. It has been pointed from reports that, after the enzymes are immobilized with the glutaraldehyde, post-modification is performed with small molecule compounds such as amino acids or NaBH₄ to increase the stability of the immobilized enzymes.

During the process of studying the immobilization of the AmDH, the inventor finds that traditional glutaraldehyde-activated amino carriers or epoxy carriers cannot well complete the immobilization of the AmDH. The obtained immobilized enzymes have the disadvantages of being low in catalytic activity and poor in reusability. Addition of protective agents such as mannitol during immobilization or small molecule post-modification of the obtained immobilized enzymes cannot overcome the above defects either.

### Summary

The present disclosure is mainly intended to provide an immobilized enzyme and an application thereof in continuous production, so as to improve the catalytic activity and reusability of an immobilized enzyme.

In order to implement the above objective, a first aspect of the present disclosure provides an immobilized enzyme. The immobilized enzyme is a Polyethyleneimine (PEI)-modified immobilized enzyme, and includes: an enzyme, the enzyme includes an Amine Dehydrogenase (AmDH) and/or a Formate Dehydrogenase (FDH); and a carrier for immobilizing the enzyme, the carrier is a cyanuric chloride-activated amino carrier.

Further, the AmDH includes an AmDH as shown in SEQ ID NO: 1. Preferably, the FDH includes a FDH as shown in SEQ ID NO: 2. Preferably, the amino carrier includes ECR8409, LX-1000HA, or LX-1000EPHA. Preferably, a molecular weight of PEI is 1.8-10 KDa.

In order to implement the above objective, a second aspect of the present disclosure provides a method for preparing an immobilized enzyme. The preparation method includes: activating an amino carrier with cyanuric chloride to obtained an activated carrier; and mixing and incubating an enzyme and the activated carrier for immobilization, and then modifying an immobilized product with PEI to obtain the immobilized enzyme. The enzyme includes AmDH and/or FDH.

Further, activating the amino carrier with the cyanuric chloride to obtain the activated carrier includes: dispersing the amino carrier in an organic solvent to obtain carrier suspension; and reacting the cyanuric chloride with the carrier suspension to activate the amino carrier, so as to obtain the activated carrier. Preferably, the organic solvent is a polar organic solvent. Preferably, the polar organic solvent includes any one or more of tetrahydrofuran, acetone, dimethyl formamide, or dimethyl sulfoxide. Preferably, a temperature for activation is 0-30°C, and more preferably, 0-5°C. Preferably, a time for activation is 2-5h. Preferably, after activation, the activated carrier is washed with the organic solvent to remove unreacted cyanuric chloride. Preferably, after washing, the method further includes drying the organic solvent on the activated carrier with nitrogen in a blowing manner. Preferably, the amino carrier is a dry carrier. Preferably, the amino carrier includes ECR8409, LX-1000HA, or LX-1000EPHA.

Further, before the enzyme and the activated carrier are mixed and incubated, the preparation method further includes washing the activated carrier with a buffer solution to replace the organic solvent. Preferably, the enzyme is prepared using the buffer solution. Preferably, the buffer solution includes a phosphate buffer solution, a glycine buffer solution, or a borax buffer solution. Preferably, a concentration of the phosphate buffer solution is 20-50mM. Preferably, a pH of the phosphate buffer solution is 6.5-9.0. Preferably, a temperature for immobilization is 0-30°C, and more preferably, 4-30°C. Preferably, the time for immobilization is 2-5h.

Further, mixing and incubating the enzyme and the activated carrier for immobilization, and then modifying the immobilized product with the PEI to obtain the immobilized enzyme includes: mixing and incubating the enzyme and the activated carrier for immobilization to obtain an initial immobilized enzyme; and post-modifying on the initial immobilized enzyme with the PEI, so as to obtain the immobilized enzyme. Preferably, the enzyme includes AmDH and/or FDH. Preferably, the AmDH includes an AmDH as shown in SEQ ID NO: 1. Preferably, the FDH includes a FDH as shown in SEQ ID NO: 2. Preferably, a PEI solution is added to the initial immobilized enzyme for post-modifying on the initial immobilized enzyme. Preferably, the PEI includes PEI. Preferably, a molecular weight of the PEI is 1.8-10 KDa. Preferably, a pH of the PEI solution is 5-8. Preferably, a final concentration of the added PEI is 0.5-3%W/V. Preferably, a temperature for post-modification is 0-30°C, and more preferably, 10-30°C. Preferably, the time for post-modification is 8-24h.

In order to implement the above objective, a third aspect of the present disclosure provides a use of the immobilized enzyme or the immobilized enzyme prepared by the preparation method in a continuous reaction.

Further, the continuous reaction includes performing the continuous reaction in a packed bed reactor. Preferably, the packed bed reactor is made by packing the immobilized enzyme into an empty chromatography column or a stainless steel chromatography column. Preferably, the volume of the packed bed reactor is 5-50 mL. Preferably, a method for packing the packed bed reactor is wet packing.

Further, the continuous reaction includes: pumping a substrate-containing reaction system into the packed bed reactor from bottom to top, and re-mixing a reaction product in the reaction system. A pump is a constant flow pump, and preferably, a pressure of the constant flow pump is less than or equal to 20 MPa. Preferably, a flow rate of the continuous reaction is 0.01-10 mL/min.

Further, the enzyme is a main enzyme, or a mixed enzyme of the main enzyme and a coenzyme. Preferably, the main enzyme is selected from the AmDH, and accordingly, the coenzyme is selected from the FDH. Preferably, the immobilized enzyme includes immobilized AmDH, or a co-immobilization system of AmDH and FDH. Preferably, the AmDH includes an AmDH as shown in SEQ ID NO: 1. Preferably, the FDH includes a FDH as shown in SEQ ID NO: 2. Preferably, the reaction system includes a substrate, or a mixture of the substrate and the coenzyme. Preferably, the immobilized enzyme is the immobilized AmDH, and the reaction system includes the mixture of the substrate and the FDH.

With the technical solutions of the present disclosure, the immobilized AmDH and FDH with high catalytic activity and good operation stability are prepared using the cyanuric chloride-activated amino carrier; and compared with an immobilized enzyme prepared using a traditional immobilization method, the immobilized enzyme has higher catalytic activity and reusability.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:

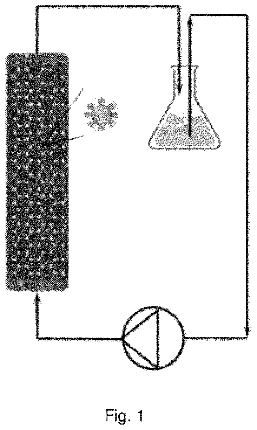

Fig. 1 is a schematic diagram of a continuous reaction apparatus of Embodiment 5 of the present disclosure.

### Detailed Description of the Embodiments

It is to be noted that the embodiments in the present application and the features in the embodiments may be combined with one another without conflict. The present disclosure will be described below in detail with reference to the embodiments.

As mentioned in the Background, traditional glutaraldehyde-activated amino carriers or epoxy carriers cannot well complete the immobilization of the AmDH. The obtained immobilized enzymes have the disadvantages of being low in catalytic activity and poor in reusability. Likewise, addition of protective agents such as mannitol during immobilization or small molecule post-modification of the obtained immobilized enzymes cannot overcome the above defects either.

Therefore, in the present application, the inventors have studied the immobilized enzyme in depth, have tried many alternatives to glutaraldehyde activation of the amino carrier, and have found that only an alternative after activation by cyanuric chloride is excellent. That is, an amino carrier activated by a cyanuric chloride carrier activating agent is found. An immobilized AmDH with high catalytic activity and good operation stability is prepared with the carrier; and compared with an immobilized AmDH prepared with a traditional immobilization method, the AmDH has higher catalytic activity and reusability. Previously, although the use of cyanuric chloride-activated resin has been reported, in the field of enzyme immobilization, only in the 1970s, there were a few literature reports on the use of cyanuric chloride-activated hydroxyl carriers, such as polysaccharide-based carriers, for enzyme immobilization, and there is no relevant report on the use of cyanuric chloride-activated amino resin for enzyme immobilization for the time being. Therefore, a series of protective solutions of the present application are proposed.

A first implementation of the present disclosure provides an immobilized enzyme. The immobilized enzyme is a PEI-modified immobilized enzyme, and includes: an enzyme, wherein the enzyme includes an AmDH and/or a FDH; and a carrier for immobilizing the enzyme, wherein the carrier is a cyanuric chloride-activated amino carrier.

Amino groups on the amino carrier can be activated by the cyanuric chloride, such that a resin structure different from traditional glutaraldehyde-activated amino resin is provided, and a new carrier is provided for enzymes that cannot be effectively immobilized on the glutaraldehyde-activated amino resin due to factors such as structures. After the enzyme is bound to the activated carrier to form an immobilized enzyme, in order to further improve the operation stability of the immobilized enzyme, the immobilized enzyme is modified by PEI, and more stable immobilization is realized with polymers to "bond" and protect the enzyme. Therefore, the catalytic activity and reusability of the immobilized enzyme are improved.

The enzyme in the immobilized enzyme is mainly targeted at a class of enzymes that are difficult to immobilize by the glutaraldehyde-activated amino carrier. Specifically, the immobilized enzyme includes an AmDH, a FDH, or a combination of other enzymes and the corresponding various enzymes. An activation principle of the cyanuric chloride-activated amino carrier is that, cyanuric chloride has three halogen active sites; during activation, one of the active sites is first used to bind to amino group, and then the remaining active sites may react with functional groups such as amino and hydroxyl on enzyme molecules to complete immobilization. Preferably, the AmDH includes, but is not limited to, AmDHs. The AmDHs in the present application refer exclusively to the AmDH of which amino acid sequence is SEQ ID NO: 1, and not to various AmDHs. Preferably, the FDH includes, but is not limited to, FDH AIY34662.1. Preferably, the amino carrier specifically activated includes, but is not limited to, LX-1000EPHA, ECR8409, or LX-1000HA. Preferably, a molecular weight of the PEI is 1.8-10 KDa.

An amino acid sequence of the AmDH is SEQ ID NO: 1 shown as follows:

MRDVFEMMDRYGHEQVIFCRHPQTGLKAIIALHNTTAGPALGGCRMIPYASTDEALED
VLRLSKGMTYKCSLADVDFGGGKMVIIGDPKKDKSPELFRVIGRFVGGLNGRFYTGTDMGT
NPEDFVHAARESKSFLGLPKSYGGGGDTSIPTALGVFHGMRATARFLWGTDQLKGRVVAIQ
GVGKVGERLLQLLVEVGAYCKIADIDSVRCEQLKEKYGDKVQLVDVNRIHKESCDIFSPCA
KGGVVNDDTIDEFRCLAIVGSANNQLVEDRHGALLQKRSICYAPDYLVNAGGIIQVADELEG
FHEERVLAKTEAIYDMVLDIFHRAKNENITTCEAADRIVMERLKKLTDIRRILLEDPRNSARR

The FDH is wild-type AIY34662.1, and an amino acid sequence thereof is SEQ ID NO: 2 shown as follows:

MKIVLVLYDAGKHAADEEKLYGCTENKLGIANWLKDQGHELITTSDKEGGNSVLDQHI
PDADIIITTPFHPAYITKERIDKAKKLKLVVVAGVGSDHIDLDYINQTGKKISVLEVTGSNVVS
VAEHVLMTMLVLVRNFVPAHEQIINHDWEVAAIAKDAYDIEGKTIATIGAGRIGYRVLERLVP
FNPKELLYYDYQALPKDAEEKVGARRVENIEELVAQADIVTINAPLHAGTKGLINKELLSKF
KKGAWLVNTARGAICVAEDVAAALESGQLRGYGGDVWFPQPAPKDHPWRDMRNKYGAG
NAMTPHYSGTTLDAQTRYAEGTKNILESFFTGKFDYRPQDIILLNGEYITKAYGKHDKK.

The AmDH refers to a class of enzymes that can catalyze the reduction of ketone compounds to chiral amine, and an inexpensive ammonium salt is used as an ammonia source, and does not rely on expensive ammonia donors. As a reduction process usually requires the delivery of hydrogen from reduced NADPH or reduced NADH, the AmDH is often used in combination with the FDH to reduce production costs. Compared with chemical-catalytic synthesis, the AmDH is not only mild in reaction condition, but also possesses higher catalytic activity, selectivity, and substrate specificity. Using the activated carrier, free enzymes, especially water-soluble free enzymes, may be immobilized to form the immobilized enzyme, such that the problems of poor stability, inability to be reused, and difficult product separation and purification are overcome. The enzyme for immobilization may be one or more enzymes for simultaneous immobilization, so as to achieve the purpose of combined use.

In a traditional method for preparing an immobilized enzyme carrier, using amino-type resin as an example, the amino-type resin usually needs to be activated by a dual-functional crosslinking agent, and then bound to the enzymes through end functional groups of the crosslinking agent. The most commonly used activation method is to activate amino by using glutaraldehyde, and then connect same with enzyme molecules using an active aldehyde group, so as to realize immobilization. It has been pointed from reports that, after the enzymes are immobilized with the glutaraldehyde, post-modification is performed with small molecule compounds such as amino acids or NaBH4 to increase the stability of the immobilized enzymes. The cyanuric chloride has an activating effect on the amino resin, making it suitable for use as an amino resin carrier, and it provides a resin structure that is different from conventional glutaraldehyde activation. After the cyanuric chloride is used to activate the amino resin, the remaining active halogen (chlorine) sites on a cyanuric chloride group may react with the enzyme molecules, and the binding capacity of different resin structures to different enzymes varies widely. The enzymes that are difficult to bind to traditional immobilized enzyme carriers may be immobilized with a carrier activating agent. The amino carrier may include various kinds of commercially available amino resins such as ECR8409 resin produced by Purolite and LX-1000HA resin produced by Xi'an Sunresin Science and Technology Company.

The PEI may be prepared into an aqueous solution with a proper concentration for use; and when being used, a final concentration may be adjusted according to factors such as the property and content of the immobilized enzyme, which may be 0.5-3%W/V. For the properties of different immobilized enzymes, the PEI with different molecular weights may be selected for modification and protection, and the molecular weight of the PEI may be 1.8-10 KDa.

A second implementation of the present disclosure provides a method for preparing an immobilized enzyme. The preparation method includes: activating an amino carrier with cyanuric chloride to obtained an activated carrier; and mixing and incubating an enzyme and the activated carrier for immobilization, and then modifying an immobilized product with PEI to obtain the immobilized enzyme; and the enzyme includes AmDH and/or FDH.

The activated amino carrier prepared by the cyanuric chloride has structures and binding sites different from traditional activated carriers, such that a new selectable carrier is provided for the preparation of the immobilized enzyme, so as to facilitate the immobilization of enzymes that are difficult to bind to traditional immobilized enzyme carriers. An enzyme and the activated carrier are immobilized at a suitable temperature, a rotary speed, etc., so as to obtain the immobilized enzyme.

In the preparation method, activating the amino carrier with the cyanuric chloride to obtain the activated carrier includes: dissolving the amino carrier in an organic solvent to obtain carrier suspension; and mixing the cyanuric chloride with the carrier suspension to activate the amino carrier, so as to obtain the activated carrier. Preferably, the organic solvent may be a polar organic solvent. Preferably, the polar organic solvent includes any one or more of tetrahydrofuran, acetone, dimethyl formamide, or dimethyl sulfoxide. Preferably, a temperature for activation is 0-30°C, and more preferably, 0-5°C. Preferably, the time for activation is 2-5h. Preferably, after activation, the activated carrier is washed by using the organic solvent to remove unreacted cyanuric chloride. Preferably, after washing, the method further includes using nitrogen to dry the activated carrier, i.e., dry the organic solvent on the activated carrier in a blowing manner. Preferably, the amino carrier is a dry carrier. Preferably, the amino carrier includes, but is not limited to, ECR8409, LX-1000HA, or LX-1000EPHA.

The amino carrier is dispersed by using the polar organic solvent such as tetrahydrofuran, acetone, dimethyl formamide, or dimethyl sulfoxide; the cyanuric chloride is used to activate the amino carrier; and according to different factors such as enzymes and carriers, the temperature for activation may be 0-30°C, and more preferably, may be 0-5°C, and the activation time is 2-5h. Low-temperature activation may protect the activity of the carrier, and may also slow down an activation rate, such that the activation rate is prevented from being too fast to generate massive heat to affect a carrier activation effect. The activation time may be properly adjusted according to conditions such as the enzymes, the carriers, or the activation temperatures, so as to generate better activation effects. The amino carrier before activation may be a dry carrier, so as to reduce compositions of liquid such as the residual water in the carrier to the reactive organic system. The residual liquid is in contact with or even wrapped the amino groups on the carrier also has an effect on activation efficiency. After activation, the same organic solvent is used to wash the activated carrier to remove unreacted cyanuric chloride. Further, the organic solvent on the activated carrier may be dried by the nitrogen in a blowing manner, so as to prevent residual cyanuric chloride and organic solvent from affecting enzyme activity in the subsequent immobilization steps, leading to adverse effect on the preparation yield and performance of the immobilized enzyme.

In the preparation method, before the enzyme and the activated carrier are mixed and incubated, i.e., before immobilization, the preparation method further includes washing the activated carrier with a buffer solution to replace the organic solvent. Preferably, the enzyme is prepared using the buffer solution. Preferably, the buffer solution includes, but is not limited to, a phosphate buffer solution, a glycine buffer solution, or a borax buffer solution. Preferably, the concentration of the phosphate buffer solution is 20-50mM. Preferably, the pH of the phosphate buffer solution is 6.5-9.0. Preferably, a temperature for immobilization is 4-30°C. Preferably, the time for immobilization is 2-5h.

Using the buffer solution to wash the activated carrier is to replace the residual organic solvent in the activated carrier, so as to prevent the residual organic solvent from affecting the enzyme activity. The enzyme is prepared using the buffer solution. The buffer solution may be a commonly-used phosphate buffer solution, a glycine buffer solution, or other commonly-used buffer solutions, and can maintain the pH of the solution relatively stable when a small amount of acid or base is added, so as to prevent products such as carbon dioxide produced by a reaction from affecting a pH value of the solution, thereby maintaining enzyme activity. The time for immobilization may be properly adjusted according to conditions such as the enzymes, the carriers, or the immobilization temperatures, so as to generate better immobilization effects.

In the preparation method, mixing and incubating the enzyme and the activated carrier for immobilization, and then modifying the immobilized product with the PEI to obtain the immobilized enzyme includes: mixing and incubating the enzyme and the activated carrier for immobilization to obtain an initial immobilized enzyme; and post-modifying on the initial immobilized enzyme with the PEI, so as to obtain the immobilized enzyme. Preferably, the enzyme includes AmDH, FDH, or one or more of other enzymes. Preferably, the AmDH includes an AmDH as shown in SEQ ID NO: 1. Preferably, the FDH includes FDH AIY34662.1 as shown in SEQ ID NO: 2. Preferably, a PEI solution is added to the initial immobilized enzyme for post-modification. Preferably, a molecular weight of the PEI is 1.8-10 KDa. Preferably, a pH of the PEI solution is 5-8. Preferably, a final concentration of the PEI is 0.5-3%W/V. Preferably, a temperature for post-modification is 10-30°C. Preferably, a time for post-modification is 8-24h.

The enzyme and the activated carrier are mixed and incubated for immobilization to obtain an initial immobilized enzyme. The enzyme used may be selected from one or more; and different enzymes may be mixed in any proportion to prepare a co-immobilization initial system of various enzymes. In order to improve the stability of the immobilized enzyme, the PEI may further be used to post-modify on the initial immobilized enzyme, and the operation stability of the immobilized enzyme is further improved by "bonding" or protecting the enzymes through polymers. A certain volume of a pre-dissolved and prepared PEI solution is added to the initial immobilized enzyme, such that efficient mixing may be realized, the final concentration of the PEI is effectively controlled, the initial immobilized enzyme and the PEI are in uniform contact with each other, and the performance of the immobilized enzyme obtained through preparation may be improved. The molecular weight of the PEI may be correspondingly adjusted according to different carriers and enzymes, so as to obtain better protective performance. The PEI may be prepared into a 10%W/V aqueous solution, and is adjusted with a 1M hydrochloric acid solution. A proper amount of the PEI aqueous solution is added to the initial immobilized enzyme to make the final concentration of the aqueous solution be 0.5-3%W/V.

A third implementation of the present disclosure provides a use of the immobilized enzyme or the immobilized enzyme prepared by the preparation method in a continuous reaction. The catalytic activity and reusability of the immobilized enzyme is greatly improved compared with an immobilized enzyme prepared by a free enzyme or a traditional process. The immobilized enzyme may be used for performing the continuous reaction in a continuous device. Compared with batch reactions, the immobilized enzyme is simple in operation and high in production efficiency.

In the application of the immobilized enzyme in the continuous reaction, the continuous reaction includes performing the continuous reaction in a packed bed reactor. Preferably, the packed bed reactor is made by packing the immobilized enzyme into an empty chromatography column or a stainless steel chromatography column. Preferably, a volume of the packed bed reactor is 5-50 mL. Preferably, a method for packing the packed bed reactor is wet packing.

The immobilized enzyme may perform the continuous reaction in the pressure-resistant and corrosion-resistant packed bed reactor such as an empty chromatography column or a stainless steel chromatography column. In order to prevent waste of the immobilized enzyme for catalysis due to overpacking and excessive pressure on the immobilized enzyme at the bottom of the packed bed reactor, affecting the mechanical structure and enzyme activity of the immobilized enzyme, the volume of the packed bed reactor may be 5-50 mL. To ensure the activity and packing uniformity of the immobilized enzyme, wet packing may be selected to pack the immobilized enzyme into the packed bed reactor.

In the above use, the continuous reaction includes pumping a substrate-containing reaction system into the packed bed reactor from bottom to top, and re-mixing a reaction product in the reaction system. A pump is a constant flow pump, and preferably, a pressure of the constant flow pump is less than or equal to 20 MPa. Preferably, a flow rate of the continuous reaction is 0.01-10 mL/min.

The substrate-containing reaction system is pumped into the packed bed reactor from bottom to top to prevent the reaction system from flow too fast in the reactor due to the effect of gravity, resulting in low conversion yield. The reaction product is re-mixed in the reaction system. For reactions with low single catalytic yield, cyclic catalysis may be performed to maintain the uniformity of the reaction system all the time, facilitating relative stability of an environment where the immobilized enzyme in the packed bed reactor is located, and the detection of the reaction system, such that raw materials such as ammonium formate are conveniently supplemented, final products are conveniently collected, and processes, required by the batch reaction, of separating the final products from the immobilized enzyme are omitted. A pump used in the continuous reaction may rationally select a medium pressure constant flow pump or a low pressure constant flow pump according to requirements of the packed bed reactor and flow rate, as well as the tolerance of the immobilized enzyme to pressure, a provided pressure range may be 0-20 MPa.

In the above use, the enzyme may be a main enzyme, or a mixed enzyme of the main enzyme and a coenzyme. Preferably, the main enzyme may be selected from the AmDH, and accordingly, the coenzyme may be selected from the FDH. The immobilized enzyme includes immobilized AmDH, or a co-immobilization system of AmDH and FDH. Preferably, the AmDH includes an AmDH as shown in SEQ ID NO: 1. Preferably, the FDH includes FDH AIY34662.1 as shown in SEQ ID NO: 2. Preferably, the reaction system includes a substrate, or a mixture of the substrate and the coenzyme. Preferably, the immobilized enzyme is the immobilized AmDH, and the reaction system includes the mixture of the substrate and the FDH.

In the use of the immobilized enzyme in the continuous reaction, the enzyme may separately be the main enzyme, or may also be the mixed enzyme of the main enzyme and the coenzyme, such that a product produced by the coenzyme is used to supply for a catalytic reaction of the main enzyme. As an AmDH reduction process generally requires the delivery of NADPH or NADH, when being used, the AmDH is used in combination with the FDH that can produce the NADH, so as to reduce production costs. The immobilized enzyme may be the separate immobilized AmDH, and in this case, the reaction needs to additionally add the FDH; and the immobilized enzyme may also be the co-immobilization system of the AmDH and the FDH, and in this case, the reaction system may be a simple substrate solution.

The beneficial effects of the present application are further described in detail below with reference to specific embodiments.

### Embodiment 1: ECR8409 immobilized AmDH

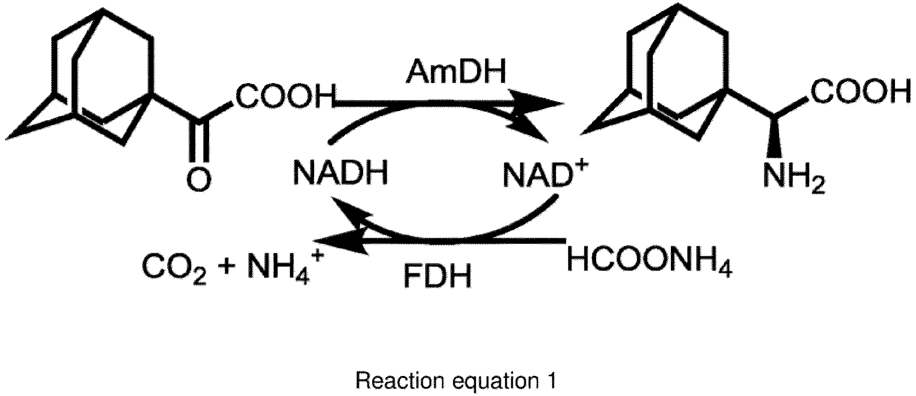

Substrate preparation: 2-(1-adamantyl)-2-oxoacetic acid shown in a reaction equation 1 was used as a substrate, 10g of the substrate was weighted to a 100mL beaker, 50mL purified water and 1.6g of K₂HPO₄·H₂O were added, and pH was adjusted to 8.0 with a 10M NaOH solution after uniform stirring; 1g of activated carbon was weighed and added to the prepared solution, and stirring was performed for 1h to adsorb impurities and pigments; and a filter paper was put on a buchner funnel, then 2.5g of diatomite was added, the purified water was added, draining was performed, and then a filter cake was obtained. Suction filtration was performed on the prepared substrate solution via the filter cake, 20mL of the purified water was added to wash the filter cake, and clear filtrate was obtained finally; 5.6g of ammonium formate and 15mg of Dithiothreitol (DTT) were weighed, and the pH was adjusted to 8.0 with the 10M NaOH solution. The purified water was added to 82 mL.

Carrier activation: 10 g of dry ECR8409 resin was taken to a four-necked bottle, 250mL of acetone was added under an ice bath condition, and stirring was performed for 0.5h. Then 5g of cyanuric chloride was added, and the reaction was continuously stirred for 3h under an ice bath. After the reaction was completed, a modified carrier was filtered, and washed for three times with the acetone for later use.

Enzyme solution preparation: a cryopreservated AmDH enzyme solution was thawed at room temperature, and then was diluted to 8.5mg/mL with a 0.1M phosphate buffer solution at pH 8.0.

Enzyme immobilization: 0.5g of the activated carrier was washed for three times with the 0.1M pH 8.0 phosphate buffer solution, 4mL of the well-prepared enzyme solution was immediately added, and immobilization was performed for 2h at 100rpm and 20°C in a shaker. Then 1mL of a prepared 10%PEI (10KDa) solution at pH 8.0 was added to continue to perform a reaction overnight. Finally, the immobilized enzyme was filtrated and washed for three times with a buffer solution, so as to obtain the immobilized AmDH.

Reaction conditions: 0.15g of the immobilized AmDH was weighed, and 0.82mL of the well-prepared substrate solution, 25µL of a 1mg/mL NAD⁺ solution, and 50µL of FDH were added. The reaction was performed for 18h in the shaker at 100rpm and 40°C. Supernatant of the reaction system was taken, and a reaction conversion rate was measured through HPLC.

Activity verification results of the immobilized AmDH were shown in Table 1. After each batch reaction was completed, the immobilized AmDH was separated by filtration, and then it was washed three times with a 0.1M phosphate buffer solution at pH 8.0.

**Table 1 Conversion rate of ECR8409 immobilized AmDH to substrate**

| Number of cycles | 1st | 2nd | 3rd | 4th | 5th |
|---|---|---|---|---|---|
| Conversion rate (%) | 99 | 99 | 95 | 93 | 91 |

### Embodiment 2: Co-immobilization of AmDH and FDH into ECR8409

A carrier activation step and enzyme solution preparation were the same as Embodiment 1; and a difference between this embodiment and Embodiment 1 lied in that, 50µL of a crude FDH AIY34662.1 enzyme solution was additionally added during enzyme immobilization. Then immobilization was completed through the same immobilization method and post-modification process.

The AmDH and the FDH were co-immobilized into ECR8409. Activity verification results were shown in Table 2. Detailed operation conditions were seen in Embodiment 1. A difference was that the crude FDH enzyme solution was no longer added to the reaction system.

**Table 2 Conversion rate of ECR8409 co-immobilized AmDH and FDH to substrate**

| Number of cycles | 1 st | 2nd | 3rd | 4th | 5th |
|---|---|---|---|---|---|
| Conversion rate (%) | 99 | 99 | 98 | 96 | 96 |

### Embodiment 3: LX-1000HA immobilized AmDH

Carrier activation: 10 g of dry LX-1000HA resin was taken to a four-necked bottle, 200mL of tetrahydrofuran was added under an ice bath condition, and stirring was performed for 0.5h. Then 2.5g of cyanuric chloride was added, and the reaction was continuously stirred for 3h under an ice bath. After the reaction was completed, a modified carrier was filtered, and washed for three times with the tetrahydrofuran for later use.

Enzyme solution preparation, immobilization process, and activity verification were the same as Embodiment 1.

Activity verification results of the immobilized AmDH were shown in Table 3.

**Table 3 Conversion rate of LX-1000HA immobilized AmDH to substrate**

| Number of cycles | 1st | 2nd | 3rd | 4th | 5th |
|---|---|---|---|---|---|
| Conversion rate (%) | 99 | 96 | 93 | 87 | 84 |

### Embodiment 4: LX-1000EPHA immobilized AmDH

Carrier activation: 10 g of dry LX-1000EPHA resin was taken to a four-necked bottle, 200mL of tetrahydrofuran was added under an ice bath condition, and stirring was performed for 0.5h. Then 2.5g of cyanuric chloride was added, and the reaction was continuously stirred for 3h under an ice bath. After the reaction was completed, a modified carrier was filtered, and washed for three times with the tetrahydrofuran for later use.

Enzyme solution preparation, immobilization process, and activity verification were the same as Embodiment 1.

Activity verification results of the immobilized AmDH were shown in Table 4.

**Table 4 Conversion rate of LX-1000EPHA immobilized AmDH to substrate**

| Number of cycles | 1st | 2nd | 3rd | 4th | 5th |
|---|---|---|---|---|---|
| Conversion rate (%) | 98 | 92 | 89 | 88 | 82 |

### Embodiment 5: Use of immobilized AmDH in continuous production

Reaction system preparation: 80mL of a substrate solution was measured, and detailed preparation steps of the substrate solution were shown in Embodiment 1. Then 2.5mg of NAD⁺ and 5mL of a crude FDH enzyme solution were added to the substrate solution. The system was placed on a magnetic stirrer to well mix, and slow stirring was maintained during a reaction process.

3.5 g of the immobilized AmDH prepared in Embodiment 1 was weighed and was packed to a 5mL empty chromatography column according to a chromatography column assembling operation flows; and the chromatography column was placed in a 40°C water bath.

A CP-M205 precise constant flow pump was connected to the reaction system, the reaction system was pumped to the chromatography column containing the immobilized enzyme at a flow rate of 0.4mL/min from bottom to top, and effluent was made to flow back to the reaction system to form a closed loop. A continuous reaction apparatus was shown in Fig. 1. After 24h of reaction, a sample of supernatant of the reaction system was sent for detection, and a conversion rate of the substrate reached 95%. Then a newly-prepared reaction system was replaced with, and was continuously put into a next cycle of reactions. Through determination, after ten cycles, the conversion rate of the reaction could still reach 89%.

Through the use of the continuous system, production operations were simpler, a processing capacity was greater, and the operation stability of the immobilized AmDH was also improved.

### Embodiment 6: Use of co-immobilized AmDH and FDH in continuous production

Reaction system preparation: 80mL of a substrate solution was measured, and detailed preparation steps of the substrate solution were shown in Embodiment 1. Then 2.5mg of NAD⁺ was added to the substrate solution. The system was placed on a magnetic stirrer to well mix, and slow stirring was maintained during a reaction process.

3.8 g of the immobilized AmDH and FDH prepared in Embodiment 2 were weighed, and were packed to a 5mL empty chromatography column according to a chromatography column assembling operation flows; and the chromatography column was placed in a 40°C water bath.

Same as Embodiment 5, the system was connected to a continuous device. After 24h of reaction, a conversion rate of the substrate reached 94%, and the conversion rate could still reach 84% after ten times of reuse.

Through the use of the continuous system, production operations were simpler, a processing capacity was greater, and the operation stability of the immobilized AmDH and FDH was also improved.

### Comparative example 1: LX-1000HA glutaraldehyde-activated immobilized AmDH

LX-1000HA glutaraldehyde activation: 1g wet weight of LX-1000HA was taken, 4mL of a 2% (w/v) glutaraldehyde solution was added, and the glutaraldehyde solution was prepared with a 20mM phosphate buffer solution at pH 7.0. Activation was performed for 1h in a 30°C shaker.

The activated carrier was washed for three times with the 0.1M phosphate buffer solution at pH 8.0, 4mL of a well-prepared enzyme solution (same as the enzyme solution prepared in Embodiment 1) was added, and immobilization was performed for 20h at 100rpm and 20°C in the shaker. Finally, the immobilized enzyme was filtrated and washed for three times with a buffer solution, so as to obtain the immobilized AmDH.

Activity verification results of the immobilized AmDH were shown in Table 5.

**Table 5 Conversion rate of LX-1000HA glutaraldehyde immobilized AmDH to substrate**

| Number of cycles | 1st | 2nd | 3rd | 4th | 5th |
|---|---|---|---|---|---|
| Conversion rate (%) | 55 | 36 | 18 | 11 | 6 |

### Comparative example 2: LX-1000HA hexamethylene diisocyanate-activated immobilized AmDH

LX-1000HA resin hexamethylene diisocyanate activation: 10 g of dry LX-1000HA resin was taken to a four-necked bottle, 200mL of N,N-dimethylformamide was added under an ice bath condition, and stirring was performed for 0.5h. Then 2.3g of hexamethylene diisocyanate was added, and the reaction was continuously stirred for 3h under an ice bath. After the reaction was completed, a modified carrier was filtered, and washed for three times with DMF for later use.

Enzyme solution preparation, immobilization process, and activity verification were the same as Embodiment 1.

Activity verification results of the immobilized AmDH were shown in Table 6.

Table 6 Conversion rate of LX-1000HA hexamethylene diisocyanate-activated immobilized AmDH to substrate.

| Number of cycles | 1st | 2nd | 3rd | 4th | 5th |
|---|---|---|---|---|---|
| Conversion rate (%) | 91 | 84 | 81 | 65 | 52 |

### Comparative example 3: LX-1000HA p-benzoquinone-activated immobilized AmDH

LX-1000HA resin p-benzoquinone activation: 1g wet weight of LX-1000HA was taken, 4mL of a 0.02g/mL p-benzoquinone ethanol solution was added, and the p-benzoquinone ethanol solution was a 20%(V/V) ethanol aqueous solution. Activation was performed for 1h in a 30°C shaker. Then the activated carrier was filtered, and washed successively by the 20%(V/V) ethanol aqueous solution and a 0.1M phosphate buffer solution at pH 8.0, so as to obtain LX-1000HA activated by p-benzoquinone.

The activated carrier was added to 4mL of a well-prepared enzyme solution (same as the enzyme solution prepared in Embodiment 1), and immobilization was performed for 20h at 100rpm and 20°C in the shaker. Finally, the immobilized enzyme was filtrated and washed for three times with a buffer solution, so as to obtain the immobilized AmDH.

Activity verification results of the immobilized AmDH were shown in Table 7.

**Table 7 Conversion rate of p-benzoquinone-activated LX-1000HA immobilized AmDH to substrate**

| Number of cycles | 1st | 2nd | 3rd | 4th | 5th |
|---|---|---|---|---|---|
| Conversion rate (%) | 37 | 19 | 11 | 9 | 2 |

### Comparative example 4: LX-1000HA immobilized AmDH (no post-modification)

A carrier activation step and enzyme solution preparation were the same as Embodiment 3. A difference lied in that a post-modification process was not performed during enzyme immobilization. Filtration was directly performed after immobilization, and then washing was performed for three times with a buffer solution, so as to obtain the initial immobilized AmDH.

Activity verification results of the immobilized AmDH were shown in Table 8.

**Table 8 Conversion rate of LX-1000HA immobilized AmDH (no post-modification) to substrate**

| Number of cycles | 1st | 2nd | 3rd | 4th | 5th |
|---|---|---|---|---|---|
| Conversion rate (%) | 98 | 63 | 27 | 20 | 13 |

### Comparative example 5: ECR8409 co-immobilized AmDH and FDH (no post-modification)

A carrier activation step and enzyme solution preparation were the same as Embodiment 2. A difference lied in that a post-modification process was not performed during enzyme immobilization. Filtration was directly performed after immobilization, and then washing was performed for three times with a buffer solution, so as to obtain an initial AmDH and FDH co-immobilized system.

Activity verification results of the immobilized AmDH were shown in Table 9.

**Table 9 Conversion rate of ECR8409 co-immobilized AmDH and FDH (no post-modification) to substrate.**

| Number of cycles | 1st | 2nd | 3rd | 4th | 5th |
|---|---|---|---|---|---|
| Conversion rate (%) | 92 | 77 | 58 | 45 | 30 |

From the above descriptions, it might be seen that, the embodiments of the present disclosure achieved the following technical effects. The immobilized AmDH and immobilized FDH with high catalytic activity and good operation stability were prepared with the cyanuric chloride-activated amino carrier; and compared with an immobilized enzyme prepared with a traditional immobilization method, the immobilized enzyme has higher catalytic activity and reusability.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure all fall within the scope of protection of the present disclosure.

## Claims

1. An immobilized enzyme, wherein the immobilized enzyme is a Polyethyleneimine (PEI)-modified immobilized enzyme, and comprises:
an enzyme, wherein the enzyme comprises an Amine Dehydrogenase (AmDH) and/or a Formate Dehydrogenase (FDH); and
a carrier, wherein the carrier is a cyanuric chloride-activated amino carrier.

2. The immobilized enzyme according to claim 1, wherein the AmDH comprises an AmDH as shown in SEQ ID NO: 1;
preferably, the FDH comprises a FDH as shown in SEQ ID NO: 2;
preferably, the amino carrier comprises ECR8409, LX-1000HA, or LX-1000EPHA; and preferably, the molecular weight of PEI is 1.8-10 KDa.

3. A method for preparing an immobilized enzyme, comprising:
activating an amino carrier with cyanuric chloride to obtain an activated carrier; and
mixing and incubating an enzyme and the activated carrier for immobilization, and then modifying the immobilized product with Polyethyleneimine (PEI) to obtain the immobilized enzyme,
wherein the enzyme comprises an Amine Dehydrogenase (AmDH) and/or a Formate Dehydrogenase (FDH).

4. The method according to claim 3, wherein activating the amino carrier with the cyanuric chloride to obtain the activated carrier comprises:
dispersing the amino carrier in an organic solvent to obtain a carrier suspension; and
reacting the cyanuric chloride with the carrier suspension to activate the amino carrier, so as to obtain the activated carrier,
preferably, the organic solvent is a polar organic solvent;
preferably, the polar organic solvent comprises any one or more of tetrahydrofuran, acetone,
dimethyl formamide, or dimethyl sulfoxide;
preferably, a temperature for activation is 0-30°C, and more preferably, 0-5°C;
preferably, a time for activation is 2-5h;
preferably, after activation, the activated carrier is washed with the organic solvent to remove unreacted cyanuric chloride;
preferably, after washing, the method further comprises drying the activated carrier with nitrogen in a blowing manner;
preferably, the amino carrier is a dry carrier; and
preferably, the amino carrier comprises ECR8409, LX-1000HA, or LX-1000EPHA.

5. The method according to claim 4, wherein before immobilization, the method further comprises washing the activated carrier with a buffer solution to replace the organic solvent,
preferably, the enzyme is prepared using the buffer solution;
preferably, the buffer solution comprises a phosphate buffer solution, a glycine buffer solution, or a borax buffer solution;
preferably, a concentration of the phosphate buffer solution is 20-50mM;
preferably, a pH of the phosphate buffer solution is 6.5-9.0;
preferably, a temperature for immobilization is 0-30°C, and more preferably, 4-30°C; and preferably, a time for immobilization is 2-5h.

6. The method according to any one of claims 3 to 5, wherein mixing and incubating the enzyme and the activated carrier for immobilization, and then modifying the immobilized product with the PEI to obtain the immobilized enzyme comprises:
mixing and incubating the enzyme and the activated carrier for immobilization to obtain an initial immobilized enzyme; and
post-modifying on the initial immobilized enzyme with the PEI, so as to obtain the immobilized enzyme,
preferably, the AmDH comprises an AmDH as shown in SEQ ID NO: 1;
preferably, the FDH comprises a FDH as shown in SEQ ID NO: 2;
preferably, post-modifying on the initial immobilized enzyme by adding a PEI solution to the initial immobilized enzyme;
preferably, a molecular weight of the PEI is 1.8-10 KDa;
preferably, a pH of the PEI solution is 5-8;
preferably, a final concentration of the added PEI is 0.5-3%W/V;
preferably, a temperature for post-modification is 0-30°C, and more preferably, 10-30°C; and
preferably, a time for post-modification is 8-24h.

7. Application of the immobilized enzyme according to claim 1 or 2, or the immobilized enzyme prepared by the method according to any one of claims 3 to 6 in a continuous reaction.

8. The application according to claim 7, wherein the continuous reaction comprises performing the continuous reaction in a packed bed reactor;
preferably, the packed bed reactor is made by packing the immobilized enzyme into an empty chromatography column or a stainless steel chromatography column;
preferably, a volume of the packed bed reactor is 5-50 mL; and
preferably, a method for packing the packed bed reactor is wet packing.

9. The application according to claim 8, wherein the continuous reaction comprises:
pumping a substrate-containing reaction system into the packed bed reactor from bottom to top, and re-mixing a reaction product in the reaction system, wherein
a pump is a constant flow pump, and preferably, a pressure of the constant flow pump is less than or equal to 20 MPa; and
preferably, a flow rate of the continuous reaction is 0.01-10 mL/min.

10. The application according to claim 9, wherein the immobilized enzyme comprises an immobilized AmDH, or a co-immobilization system of AmDH and FDH;
preferably, the AmDH comprises an AmDH as shown in SEQ ID NO: 1;
preferably, the FDH comprises a FDH as shown in SEQ ID NO: 2;
preferably, the reaction system comprises a substrate or comprises a mixture of the substrate and the FDH; and
preferably, the immobilized enzyme is the immobilized AmDH, and the reaction system comprises the mixture of the substrate and the FDH.
